# EUROPÄISCHE PATENTSCHRIFT

(11) **EP 1 791 601 B1**
(45) Veröffentlichungstag und Bekanntmachung des Hinweises auf die Patenterteilung: **01.12.2010**
(21) Anmeldenummer: 05786111.4
(22) Anmeldetag: 23.09.2005
(51) Int. Cl.: A61P 31/10

(54) **NEUER IMPFSTOFF FÜR VETERINÄRE UND HUMANMEDIZINISCHE PROPHYLAXE UND THERAPIE**
NOVEL VACCINE FOR VETERINARY AND HUMAN MEDICINE PROPHYLAXIS AND THERAPY
NOUVEAU VACCIN UTILISE A DES FINS DE PROPHYLAXIE ET DE TRAITEMENT EN MEDECINE VETERINAIRE ET HUMAINE

(30) Priorität: 24.09.2004 DE 102004046391
(43) Veröffentlichungstag der Anmeldung: 06.06.2007
(73) Patentinhaber: Bioveta AG, 683 23 Ivanovice na Hané (CZ); VRZAL, Vladimir, 683 23 Ivanovice na Hane (CZ); BITTNER, Libor, 682 01 Vyskov (CZ)
(72) Erfinder: BRAUN, Dagmar, 17493 Greifswald - Insel Riems (DE); VRZAL, Vladimir, 683 23 Ivanovice na Hané (CZ); BITTNER, Libor, 682 01 Vyskov (CZ); KOUKALOVA, Dagmar, 772 00 Olomouc (CZ)
(74) Vertreter: Jeck, Anton
(86) Internationale Anmeldenummer: PCT/EP2005/010347
(87) Internationale Veröffentlichungsnummer: WO 2006/032533

(56) Entgegenhaltungen:
- WO-A-02/32442
- CS-B- 277 558
- US-A1- 2003 039 667
- KOUKALOVA DAGMAR ET AL: "Experimental nonspecific immunostimulation by the Propionibacterium acnes vaccine" ACTA UNIVERSITATIS PALACKIANAE OLOMUCENSIS FACULTATIS MEDICAE, Bd. 133, Nr. 0, 1992, Seiten 19-23, XP009058175 ISSN: 0301-2514
- DATABASE BIOSIS [Online] BIOSCIENCES INFORMATION SERVICE, PHILADELPHIA, PA, US; 2001, NALEWAK L ET AL: "Media evaluation for the detection of anaerobic microorganism in biopharmaceutical manufacturing samples" XP002359351 Database accession no. PREV200200201215 & ABSTRACTS OF THE GENERAL MEETING OF THE AMERICAN SOCIETY FOR MICROBIOLOGY, Bd. 101, 2001, Seiten 419-420, 101ST GENERAL MEETING OF THE AMERICAN SOCIETY FOR MICROBIOLOGY; ORLANDO, FL, USA; MAY 20-24, 2001 ISSN: 1060-2011

## Beschreibung

Die vorliegende Erfindung betrifft einen neuen Impfstoff, seine Verwendung zur Immunoprophylaxe und Behandlung von Candidamykosen in der Human und Veterinärmedizin sowie Verfahren zu seiner Herstellung.

### Stand der Technik

Mykosen werden durch Hefen und niedere Pilze verursacht. Von den ungefähr 10.000 bekannten Arten sind etwa nur 300 für den Menschen pathogen. Ihre schädigende Wirkung beruht einerseits auf dem direkten Angriff auf das lebenden Gewebe und andererseits auf der Produktion von Mykotoxinen und deren indirekten Wirkung, wie der Auslösung von Allergien. Abhängig von dem Ort des Befalls unterscheidet man zwischen systemischen und oberflächlichen Mykosen. *Candida* ist eine Gattung asporogener Sprosspilze mit zahlreichen fakultativ pathogenen Arten. Zu den häufigsten Erregern der Candidiasis zählen *C*. *albicans, C. guilliermondii, C. krusei, C. parapsilosis, C. pseudotropicalis, C. pulcherrima, C. stellatoidea, C. glabrata* und *C*. *tropicalis.*

Candidamykosen sind durch Candida-Arten, meist *Candida albicans,* hervorgerufene opportunistische Mykosen (Pilzerkrankungeh), die als kutane oder mukokutane Erkrankung insbesondere in Form von Stomatitis (Soor), Osophagitis, Windelerythem oder Vulvovaginitis auftreten können. Sie können aber auch systemisch als lebensbedrohliche generalisierte Kandidose, insbesondere bei Neugeborenen sowie Patienten mit gestörter Immunkompetenz, auftreten. Dies geschieht v.a bei Zytostatika- oder Antiblotikagabe, bei Gabe von Steroiden oder Hormonen, bei parenteraler Ernährung, malignen Erkrankungen, Endokrinopathien oder Immundefekten. In letzter Zeit ist die Häufigkeit von durch *Candida* hervorgerufenen nosocomialen Mykosen stark angestiegen (siehe z.B. Dole 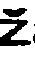 zal, 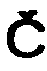 eska a Slovenska farmacie, Band LI, Ausgabe 5, September 2002, S. 226-235 und stellen wichtige Ursachen für die für Morbidität und Mortalität von insbesondere Krankenhauspatienten dar.

Während es zur Behandlung oberflächlicher Mykosen eine breite Auswahl von Wirkstoffen gibt, sind die Möglichkeiten zur Behandlung von systemischen Mykosen sehr beschränkt.

Die Rolle, die die Gentechnik bei der Entwicklung neuer Therapeutika gegen Pilzinfektionen spielt ist im Artikel von Korabe 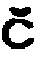 na et al., Epidemiol. Microbiol. Immunol., 52, 2003, N. 1, S. 25-33 zusammengefasst.

Wie schon oben beschrieben ist in den letzten Jahren die Häufigkeit der Pilzinfektionen dramatisch angestiegen. Dies hängt insbesondere mit der steigenden Anzahl von Patienten mit einem suppremierten Immunsystem wie Transplantatempfängern, Krebspatienten und AIDS-Patienten zusammen. Andererseits entstanden durch die weitverbreitete Anwendung von Breitsprektrumantimykotika eine Vielzahl von resistenten Erregerstämmen was weiter zur Verschärfung der Situation beiträgt (siehe z.B. Jarvis et al., Clinical Infectious Disease, 1995, 20, S. 1526-30; Beck-Sague et al., The Journal of Infectious Disease, 1993, 167, S.1247-51; Gottfredson set al., Pathology, 30, 1998, S. 405-418; Tom 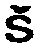 ikova' et al., Epidemiol. Microbiol. Immunol., 51, 2002, Nr.3, S. 119-124; Rex et al., Antimicrob. Agents Chemother., 39, 1995, S. 1-8; Kunova', Epidemiol. Microbiol. Immunol., 51, 2002, Nr. 3, S. 131-134).

Die am meisten verbreitete Pilzerkrankung im Menschen ist allerdings eine Pilzinfektion der Vagina, hierbei ist es ein Problem, dass solche Infektionen sehr oft chronisch werden und in diese Fällen Antimykotika die lokal und meist auch über einen langen Zeitraum gegeben werden oft wirkungslos bleiben.

Mit der Bereitstellung von Therapeutika gegen Mykosen auf immunologischer Basis beschäftigen sich unter anderem de Bernardis et al., Infection and Immunity, Feb. 1994, S. 509-519; de Bernardis et al., Infection and Immunity, Aug. 1997, S. 3399-3405; de Bernardis et al., Infection and Immunity, Juni 2000, S. 3297- 3304; Martinez et al., Clinical Microbiology Reviews, Jan. 1998., S. 121-141; Polonelli et al., Med. Mycol., 2000, 38 Suppl. 1: 281-292; Medling et al., Mycoses, 1996, 39, S. 177-183 und Odds F.C. in Candida and Candidosis; 2. Aufl. 1988, Baillière Tindall W.B. Saunders, London.

Daher gibt es schon seit einiger Zeit intensive Bestrebungen einen Impfstoff gegen fungale Infektionen, insbesondere gegen Candidainfektionen zu entwickeln. Die Ansätze sind vielfältig.

So beschreibt CS 277 558 einen Impfstoff zur peroralen und/oder lokalen Behandlung von durch Hefen verursachter chronischer Vaginitis und anderer chronischer Schleimhautentzündungen welcher auch in Kombination mit Antimykotika verwendet werden kann. Dieser Impfstoff enthält 3 *Candida albicans* Stämme (CA 37, CA 91 und CA 120), einen *Candida krusei* Stamm (CK 9), einen *Candida glabarata*-Stamm (TG 15) und 3 *Propionibacterium acnes* Stämme (PA 3, PA 17 und PA 530).

In RU 2185842 wird eine Zubereitung aus *Bacillus subtillis* gegebenenfalls in Kombination mit *Bifidobacterium bifidum* und/oder *Lactobacilli* Stämmen zur Behandlung von Urogenitalinfektionen welche u.a. durch *Candida* verursacht werden beschrieben.

US 5,578,309, US 2002/0160009A1 und WO 00/52053 beschreiben die Verwendung von Phosphomannan aus *Candida albicans* zur Therapie von Candidainfektionen und u.a. auch die Verwendung von monoklonalen Antikörpern zur passiven Immunisierung gegen Candidainfektionen.

Ein enteral zu applizierender Impfstoff ohne Adjuvans der abgetötete Mikroorganismen enthält welche die Vagina infizieren können, wie z.B. *Candida albicans, Gardnerella vaginalis, Neisseria gonorrhoea, Trichomonas vaginalis* oder *Herpes genitalis* wird in WO 96/07426 offenbart.

US 6,099,853 beschreibt u.a. eine Formulierung zur Behandlung von Urogenitalinfektionen in Form eines Suppositoriums welches 8 bis 14 verschiedene inaktivierte uropathogene Bakterienstämme der Spezies *Escherichia coli, Klebsiella pneumoniae, Proteus mirabilis, Proteus morganii* und *Streptcoccus faecalis* enthält.

Antikörper oder antigenenthaltende Mikropartikel zur passiven oder aktiven Immunisierung des weiblichen Genitaltraktes sind Gegenstand von US 4,732,763. US 5,288,639 beschreibt die Verwendung einer Polypeptidsequenz aus *Candida* mit hoher Homologie zu bekannten Stressproteinen anderer Organismen und damit erzeugter Antikörper zur Therapie und Diagnose von Mykosen, insbesondere von Candidamykosen.

US 4,678,748 wiederum offenbart ein Verfahren zur Herstellung von immunobiologischen Zubereitungen zur Diagnose, Prophylaxe und/oder Therapie von *Candida guilliermondii* Infektionen.

### Detaillierte Beschreibung der Erfindung

Die vorliegende Erfindung betrifft zunächst einen neuen Impfstoff enthaltend eine Kombination der *Candida* Stämme
a1) *Candida albicans* CCM 8355
a2) *Candida glabrata* CCM 8356 und
a3) *Candida krusei* CCM 8357 und
a4) dem immunmodulierenden *Propionibacterium acnes* -Stamm Propionibacterium acnes CCM 7083 und gegebenenfalls einem oder mehrer Hilfsstoffe wie Vehikel, Füllmittel oder Träger,
wobei das Verhältnis der Bestandteile a1 bis a4 im Endprodukt a1:a2:a3:a4 = 10-20: 10-20: 10-20:40-70 beträgt.

Ferner betrifft die vorliegender Erfindung Verfahren zur Herstellung der erfindungsgemäßen Impfstoffe.

Die erfindungsgemäßen Impfstoffe finden sowohl in der Human- als auch in der Veterinärmedizin ihre Verwendung.

Erfindungsgemäß wird unter Candidamykosen (Candidiasis) eine durch Pilze der Gattung *Candida* verursachte Erkrankung verstanden. Sie kann wie bereits oben beschrieben sowohl lokal als auch systemisch auftreten.

Somit stellt die vorliegende Erfindung einen Impfstoff zur Prophylaxe und/oder Therapie von lokaler, kutaner oder mukokutaner, und/ oder systemischer Candidiasis bereit.

Hierbei kann die lokale Candidiasis die äußere Mucosa des Genitaltraktes, den Urogenitaltrakt, die Mundhöhle, den Gastrointestinaltrakt, die Brustdrüsen, den Gehörgang oder die Haut betreffen.

Insbesondere sind hiervon Stomatitis (Soor), Ösophagitis, Windelerythem, Pyodermitis pustulosa, Folliculitis, erthematosquamose Form, Intertrigo, Phlebitis, Candidosis granulomatosa, Granuloma, Onychia, Paronychia, Erosio interdigitalis candidamycetica, Maduramykose (insbesondere der Beine), Vulvovaginitis, Abszesse nach Injektionen, Komplikationen nach Verbrennungen, Cheilitis angularis (Perleche), Glossitis, Lingua pilosa nigra, Hyperplastische Zungencandidiasis, orale Leukoplakie, Tonsillencandidiasis, Keratitis, Endolphthalmitis, Ulcus cornea, Otitis und Balanitis umfasst.

Unter die systemische Candidiasis die mit dem erfindungsgemässen Impfstoff behandelt werden kann bzw. gegen die mit dem erfindungsgemässen Impfstoff vorgebeugt werden kann fallen unter anderem generalisierter systemische Candidose, Candidämie, akute hämatogene disseminierte Candidiasis, chronisch disseminierte Candidiasis, Candida-Endokarditis, Candida-Perikarditis, eitrige Phlebitis, Candida-Meningitis, Candida-Pneumonie, Candida-Osteomyelitis, Candida-Mediastinitis, Candida-Arthritis, Candida-Gastritis, Candida-Colitis, Interstitielle Soormykose, Peritonitis, Angiolitis, oropharyngeale und ösophageale Candidiasis, renale Moniliasis, Uretritis, Cystitis pseudomembranacea, Myzetom, Nierenabszess, Candida-Bronchitis, Candida-Bronchopneumonie, primäre bronchiale Candidiasis, primäre Lungen-Candidiasis, Candida-Meningoencephalitis, Gehirnabzesse oder Schädigung der Augen auf Grund von disseminierter primärer systemischer Candidiasis. Ferner auch allergiebedingte Erkrankungen wie Bronchialasthma, Bronchitis, Rhinitis, Ekzeme, Farmerlunge und ähnliche Syndrome.

Der immunmodulierende *Propionibacterium acnes* Stamm ist *Propionibacterium acnes* CCM 7083. Weitere immunmodulierende *Propionibacterium acnes* -Stämme die getestet wurden sind *Propionibacterium acnes* PA3 *und Propionibacterium acnes* PA 530.

Weiterhin ist ein Impfstoff bevorzugt bei welchen das Verhältnis a1:a2:a3:a4 = 15-20:15-20:15-20:40-55 ist oder 10-15:10-15:10-15:55-70 ist. Insbesondere bevorzugt sind Verhältnisse a1:a2:a3:a4 = 10:10:10:70 bzw. 20:20:20:40 oder 15:15:15:55 bevorzugt. Ganz besonders wird hierbei ein Verhältnis von a1:a2:a3:a4 = 10:10:10:70 zur oralen Applikation, ein Verhältnis von a1:a2:a3:a4 = 15:15:15:55 zur vaginalen Applikation und ein Verhältnis von a1:a2:a3:a4 = 20:20:20:40 zur rektalen Applikation bevorzugt.

Der erfindungsgemäße Impfstoff enthält vorzugsweise 2 bis 10 mg Gesamttrockengewicht der Impfstämme a1 bis a4 pro Dosis.

Der Gesamtgehalt an Formaldehyd im Endprodukt beträgt vorzugsweise unter 0,02 Gew. %.

Die erfindungsgemäßen Impfstoffe können vom Fachmann in an sich bekannter Weise zu Darreichungsformen zur parenteralen, lokalen oder oralen Applikation formuliert werden z.B. gemäß Remington's Pharmaceutical Sciences, 15. Auflage.

Insbesondere bevorzugt ist hierbei die topische, vaginale oder rektale Applikation.

Darreichungsformen des erfindungsgemäßen Impfstoffes umfassen insbesondere (Hart)Kapseln, Tabletten, Lutschtabletten, Pastillen, Sirupe, orale Suspensionen, orale Emulsionen, Globuli, Pillen, Rektalsuppositorien, Vaginalsuppositorien, Vaginalovula, Ampullen, Fertigspritzen, Aerosole, Insufflationen und Mundwasser.

Erfindungsgemäß verwendete Hilfsstoffe umfassen z.B. Verdünnungsmittel, Träger, Vehikel, Konservierungsmittel, Farbstoffe, Sprengmittel, Bindemittel, Emulgatoren, Solubilisatoren, Netzmittel, Lösungsmittel, Puffersubstanzen, Gelbildner, Verdickungsmittel, Filmbildner, Gleitmittel, Schmiermittel, Formentrennmittel, Fließregulierungsmittel, Sorptionsmittel, Antioxidantien und Geschmack- und Geruchskorrigentien.

Grundlagen für Suppositorien und Vaginalovula schließen lipidhaltige und wasserlösliche Zubereitungen mit ein, hierbei werden insbesondere Kakaobutter, Hartfett, Macrogol 6000, PEG 6000 oder Mischungen von Macrogolen oder Glycerol-Gelatine verwendet.

Als Träger/Vehikel bei der Formulierung von Darreichungsformen zur oralen Applikation werden insbesondere Aerosil, Saccharose, Stärke, insbesondere Kartoffelstärke oder Mischungen von mindestens zwei der vorher genannten verwendet.

Der erfindungsgemäße Impfstoff kann sowohl in der Humanmedizin als auch der Vetrinärrnedizin eingesetzt werden und dient der Prophylaxe und/oder Therapie von lokaler oder systemischer Candidiasis, wie z.B. Dermato-, Pneumo-, Enteromykosen oder tiefe Mykosen wie Chromo-, Madura-, Keloid-Blasto-, Phyko, Blasto-, oder Kokzioidomykose.

Durch die breite Zusammensetzung bezüglich der Antigene ist der erfindungsgemäße Impfstoff in der Lage nicht nur gegen homologe *Candida-*Stämme eine Immunität zu erzeugen, sondern auch eine Kreuzreaktion gegenüber anderen Erregern von Mykosen aufzubauen, wie z.B. Sprosspilzen wie *Cryptococcus neoformans,* Schimmelpizen wie *Aspergillus flavus, Aspergilllus parasiticus, Aspergillus niger, Aspergillus fumigatus, Aspergillus terreus, Fusarium oxysporum, Mucor plumbeus, Mucor rouxii, Absidia corymbifera, Emericella nidulans, Alternaria solani, Alternaria alternate, Malassezia pachydermatis, Malassezia furfur, Saccharomyces cerevisiae* und *Rhodotorula rubra;* dimorphen Pilzen wie *Candida dubliniensis, Candida lipolytica, Candida zeylanoides, Candida pelliculosa, Candida lusitaniae, Candida kefyr, Candida parapsilosis, Candida tropicalis* und *Candida guilliermondii* und Dermatophyten wie *Trichophyton verrucosum, Trichophyton rubrum, Trichophyton mentagrophytes, Microsporum canis* und *Trichophyton equinum.* und somit Schutz zu gewährleisten

Der erfindungsgemäße *Propionibacterium acnes* Stamm hat eine signifikante unspezifische immunstimulierende Wirkung und hat somit einen positiven Effekt auf die Ausbildung der spezifischen Immunantwort und bewirkt insgesamt eine Verstärkung der Immunantwort.

Ferner kann der erfindungsgemäße Impfstoff auch in Kombination mit anderen Arzneimitteln wie Antimykotika oder Antibiotika verabreicht werden, dies kann simultan, in einem gewissen zeitlichen Abstand nacheinander oder als spezielles Therapieschemata erfolgen.

Die verwendeten Impfstämme a1 bis a3 gehören der Gattung *Candida* an, Kulturen der Stämme *Candida albicans,* CCM 8355 (a1), *Candida glabrata*; CCM 8356 (a2) und *Candida krusei,* CCM 8357 (a3) wurden von den Anmeldern bei der CCM - Czech Collection of Microorganisms, Masaryk Univertität, Tvrdeho 14, 620 00 Brno; Tschechische Republik am 23. Januar 2003 nach dem Budapester Vertrag unter den Hinterlegungsnummern CCM 8355, CCM 8356 und CCM 8357 hinterlegt.

Verwendet wird *Propionibacterium acnes*, CCM 7083 als Impfstamm a4, dem immunomodulierenden *Propionibacterium acnes* Stamm. Kulturen von *Propionibacterium acnes* CCM 7083 wurden ebenfalls von den Anmeldern bei der CCM - Czech Collection of Microorganisms, Masaryk Univertität, Tvrdeho 14, 620 00 Brno, Tschechische Republik am 23. Januar 2003 nach dem Budapester Vertrag unter der Hinterlegungsnummer CCM 7083 hinterlegt.

Durch die Auswahl der Candida-Stämme und des immunmodulierenden *Propionibacterium* acnes-Stammes im erfindungsgemäß optimierten Mischungsverhältnis haben die erfindungsgemäßen Impfstoffe ein positiven Effekt auf das Immunsystem des Patienten und können sowohl zur Prophylaxe als auch zur Therapie einer bestehenden lokalen oder systemischen Candidamykose angewandt werden.

Denn obwohl eine sehr große Anzahl von pathogenen *Candida-* und *Propionibacterium*-Stämmen bekannt ist konnte im Allgemeinen mit der Ausnahme weniger Stämme keine immunmodulierende oder immunstimulierende Wirkung in verschiedenen in vivo Tiermodellen beobachtet werden. Überraschenderweise zeigten jedoch die erfindungsgemäßen Impfstämme einen positiven Effekt auf das Immunsystem.

Die Stämme werden wie folgt weiter charakterisiert:

### 1.1 Candida-Stämme

### a) Assimilierung

| Test-assimilierung von | | | |
|---|---|---|---|
| | *Candida albicans* CCM 8355 | *Candida krusei* CCM 8357 | *Candida glabrata* CCM 8356 |
| Galactose | + | - | - |
| Maltose | + | - | - |
| Saccharose | + | - | - |
| Xylase | + | - | - |
| Adonitol | + | - | - |
| Citrat | + | + | - |
| Erythritol | + | - | - |
| Mannitol | + | - | - |
| Sorbase | - | - | + |
| Amylum | + | - | - |
| Trehalose | + | - | + |

### b) Fermentationsaktivität

| Test-fermentation von | | | |
|---|---|---|---|
| | *Candida albicans* CCM 8355 | *Candida krusei* CCM 8357 | *Candida glabrata* CCM 8356 |
| Galactose | - | - | - |
| Maltose | + | - | - |

### c) Weitere Charakterisierung der Stämme

| Test | | | |
|---|---|---|---|
| | *Candida albicans* CCM 8355 | *Candida krusei* CCM 8357 | *Candida glabrata* CCM 8356 |
| Bildung von | | | |
| Chlamydosporen | + | - | - |
| Pseudomycelium | + | + | - |
| Resistenz gegenüber Actidion | + | - | - |
| (cycloheximid) NalO₄ | + | - | + |
| Toleranz gegenüber | | | |
| pH 1,55 | + | + | - |
| pH 1,4 | + | - | - |
| Wachstum auf McConkey Agar | - | + | + |
| Serotyp | A | Nicht bestimmt | Nicht bestimmt |

Alle ausgewählten Candida-Stämme wuchsen bei 37°C und waren resistent gegenüber Natriumchlorid und Borsäure. Die Stämme bildeten weder Artrosporen oder Kapseln, noch Pigmente, Fenoloxidase oder Proteinase.

### 1.2 Propionibacterium-Stamm

| Test | |
|---|---|
| | *Propionibacteri um acnes* CCM 7083 |
| Abbau von | |
| Fructose | - |
| Galactose | - |
| Glucose | + |
| Hydrolyse von Gelatine | - |
| Koagulation von Milch | - |
| Reduktion von Nitraten | + |

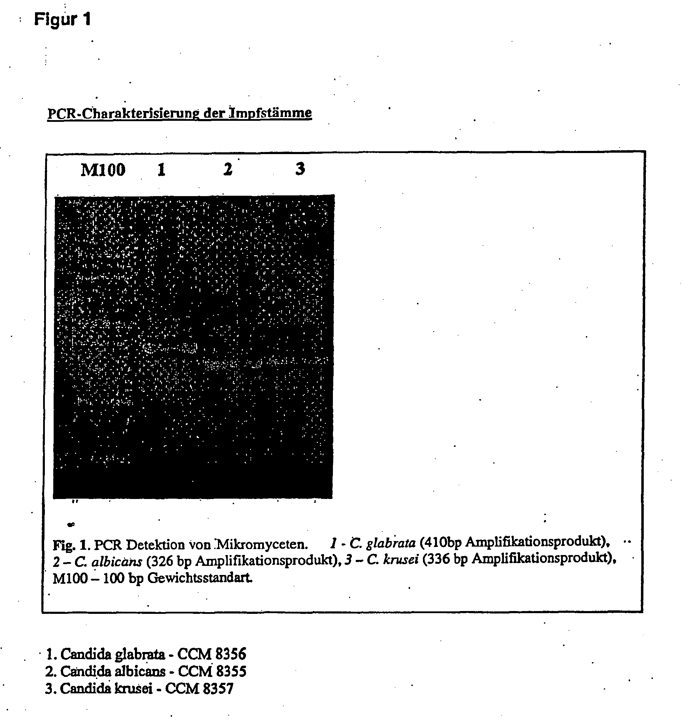

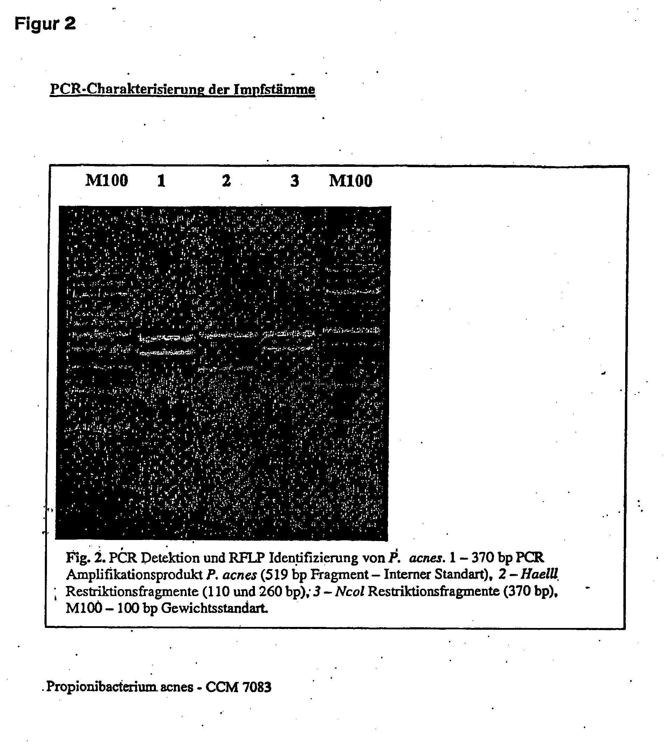

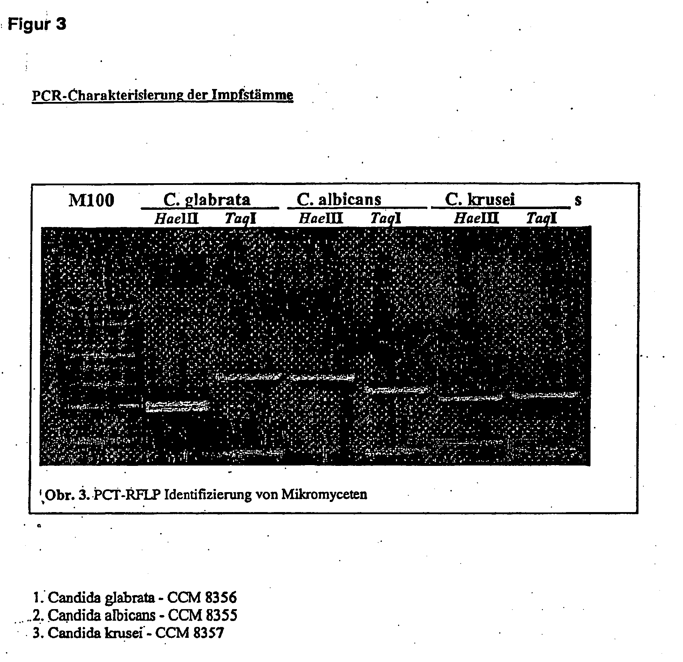

Der ausgewählte Propionibakterium-Stamm war Katalase positiv (d.h. produzierte Katalase), empfindlich gegenüber Penicilin und bildete Indol. Der Stamm war immobil und baute weder Lactose, Maltose, Mannitol, Rhamnose, Saccharose, Salicin, Trehalose oder Esculin ab. Der Stamm bildete weder Lecitinase, Lipase, Urease noch Pigment. Er zeigte auch keine Vorverdauung von Milch. Figuren 1-3 zeigen die PCR-Charakterisierung der 4 Impfstämme.

Ferner betrifft die vorliegende Erfindung ein Verfahren zur Herstellung des erfindungsgemäßen Impfstoffes umfassend die folgenden Schritte:
1) getrenntes Heranzüchten der Impfstämme a1 bis a4 und Isolierung der Zellmasse;
***2)*** Inaktivieren der Candida-Stämme a1 bis a3 mittels Formaldehyd
***3)*** Hitzeinaktierung des *Propionibacterium* acnes-Stammes a4
***4)*** getrennte Lyophilisation der Stämme a1 bis a4; und
5) Vermischen der Impfstämme mit gegebenenfalls einem oder mehreren Hilfsstoffen, Trägern, Vehikel; und
***6)*** gegebenenfalls Formulierung einer Darreichungsform
wobei Schritt 1), wie in Anspruch 18 spezifiziert ausgeführt wird.

Weitere mögliche Verfahren zur Inaktivierung der Impfstämme sind unter anderem die chemische Inaktivierung wie z.B. mit Betapropiolacton.

In einem erfindungsgemäßen Verfahren werden in Schritt 1) die *Candida* Stämme a1 bis a3 getrennt auf Glucosopepton Agar bei 23 bis 27°C für 48 bis 72 Stunden oder Sabouraud Agar bei 35 bis 39°C für 22 bis 27 Stunden unter aeroben Bedingungen angezüchtet isoliert und nachfolgend mit sterilem Wasser zur Injektion gewaschen und durch drei Gefrier- und Tauzyklen aufgebrochen.

In einem weiteren bevorzugten erfindungsgemäßen Verfahren werden die *Candida* -Stämme a1 bis a3 in Schritt 1) getrennt in Sabouraud Medium bei 37°C für 24 bis 48 Stunden unter aeroben Bedingungen herangezüchtet.

In einem weiteren erfindungsgemäßen Verfahren werden die *Candida* -Stämme a1 bis a3 in Schritt 1) getrennt in Sabouraud Medium bei 37°C für 24 bis 48 Stunden unter aeroben Bedingungen herangezüchtet. Danach werden die Bakterienzellen unter Verwendung einer Ultrafiltrationspatrone mit einem Cut-off von 300 kDa isoliert und durch wiederholtes Waschen mit steriler physiologischer Kochsalzlösung und Zentrifugieren bei 4500g (z.B. Jouan KR22, 5000 UPM) gereinigt. Die isolierten Bakterienzellen werden daraufhin mit sterilem Wasser zur Injektion gewaschen und durch drei Gefrier-Tauzyklen desintegriert (mindestens -20°C). Statt der Desintegration der Impfstämme a1 bis a3 mittels Gefrier-Tauzyklen ist auch die Desintegration durch Ultraschall möglich.

In einem erfindungsgemäßen Verfahren wird in Schritt 1) der *Propionibacterium acnes*-Stamm a4 auf einem Blutagar, einem Reinforced-Closstridium Agar oder in einem VL Agar mit Blut bei 35 bis 39°C für 46 bis 50 Stunden unter streng anaeroben Bedingungen angezüchtet, isoliert und nachfolgend mit sterilem Wasser zur Injektion gewaschen und lysiert, bei 2 bis 8°C für 22 bis 26 Stunden extrahiert und in Schritt 3) durch dreimaliges Erhitzen auf 56 bis 62°C für eine Stunde mit einem Abstand von mindestens 24 Stunden inaktiviert.

Nach einem weiteren erfindungsgemäßen Verfahren wird in Schritt 1) der *Propionibacterium acnes* -Stamm a4 in Reinforced-Clostridial-Medium bei 35 bis 39°C für 46 bis 50 Stunden unter streng anaeroben Bedingungen unter statischen Bedingungen herangezüchtet und die Bakterienzellen unter Verwendung einer Ultrafiltrationspatrone mit einem Cut-off von 300kDa isoliert. Danach folgt eine Reinigung durch wiederholtes Waschen mit physiologischer Kochsalzlösung und Zentrifugation bei 4500 g (z.B. Jouan KR 22, 5000 UPM) gefolgt von Lyse mit sterilem Wasser zur Injektion und Extraktion bei 2 bis 8°C für 22 bis 26 Stunden. In Schritt 3) wird das bakterielle Material durch dreimaliges Erhitzen auf 56 bis 62°C für eine Stunde mit einem Abstand von mindestens 24 Stunden inaktiviert.

In einem ganz besonders bevorzugten erfindungsgemäßen Verfahren werden in Schritt 1) die Stämme a1 bis a4 getrennt in einem Fermenter/Bioreaktor, bevorzugt kontinuierlich, angezüchtet und nachfolgend isoliert. Ein erfindungsgemäß verwendbarer Bioreaktor ist z.B. APPLIKON ADI, 270 Liter. Hierbei ist es bevorzugt, dass die *Candida*-Stämme a1 bis a3 in einem sterilen flüssigen Sabouraud-Medium unter aeroben Bedingungen, bei 0.1-0.2 bar, bei 15-20% O₂, bei einem pH-Wert von 5,6 bis 7,2 für eine Dauer von 16 bis 24 Stunden bei 23°C bis 27°C bei 40-50 Umdrehungen pro Minute kultiviert werden.

Bevorzugte Kultivierungsbedingungen in einem Bioreaktor/Fermenter für den *Propionibacterium acnes* Stamm a4 ist eine Kultivierung bei anaeroben Bedingungen, bei 0.1-0.2 bar unter Zugabe einer Mischung von N:CO₂ (1:2) von ca. 10 I/min, bei max. 1 % O₂ bei einem pH-Wert von 6,4 bis 7,2 für eine Dauer von 15 bis 17 Stunden bei 35°C bis 39°C in einem flüssigen modifizierten Reinforced-Clostridium-Medium bei 40 bis 50 Umdrehungen pro Minute.

Die Isolation der Zellmasse bei Anzucht im Fermenter/Bioreaktor erfolgt bevorzugt durch industrielle Ultrazentrifugation bei 3600 g bis 5300 g oder Ultrafiltration.

Weitere mögliche Verfahren zur Inaktivierung der Impfstämme sind unter anderem die chemische Inaktivierung wie z.B. mit Betapropiolacton.

Wie aus den untenstehenden Anwendungsbeispielen im Tiermodell und in der Humanmedizin deutlich hervorgeht stellt die vorliegende Erfindung einen äußerst wirksamen Impfstoff bereit, der zudem leicht appliziert werden kann, da eine parenterale Verabreichung nicht notwendig ist.

### Herstellungs- und Anwendungsbeispiele

### Herstellungsbeispiel 1: Herstellung eines Impfstoffes zur oralen Applikation

Zur Anzucht der erfindungsgemäßen *Candida*-Stämme a1 bis a3 wurde Sabouraud Agar verwendet der bei einem Überdruck von 80 bis 100 kPa für 20 bis 30 min sterilisiert worden war. Nachdem der pH-Wert auf pH 6,0 bis 6,9 eingestellt worden war wurde der Agar in Glaspetrischalen gegossen und nochmals bei den selben Bedingungen sterilisiert. Die Petrischalen mit dem verfestigten Nährboden wurden auf Sterilität überprüft und mit den einzelnen *Candida*-Impfstämmen a1 bis a3 getrennt inokuliert. Hierzu wurden Lyophilisate oder frische Inokula verwendet welche auf der Oberfläche der Nährböden verteilt wurden und nachfolgend bei 23 bis 27°C für 48 bis 72 Stunden unter aeroben Bedingungen bei Normaldruck und einer Luftfeuchtigkeit von 90-98% kultiviert bis ein optimales Wachstum erreicht wurde (bestimmt durch OD-Messung bei 605 nm unter Verwendung eines Spektrometers in Br). Die Kulturen wurden dann mit sterilem Wasser zur Injektion von der Oberfläche der Nährböden geerntet und durch drei Gefrier- und Tauzyklen (mindestens -20°C) desintegriert. Das erhaltene Lysat wurde mittels Zugabe von wässriger Formaldehydlösung inaktiviert. Der Gesamtformaldehydgehalt des Endproduktes war hierbei unter 0,02 Gew%. Nach einem Test auf Sterilität wurde das inaktivierte Lysat der *Candida*-Stämme für 48 Stunden lyophilisiert bis der Restwassergehalt bevorzugt unter 3% lag.

Zur Anzucht des *Propionibacterium* acnes-Impfstamm wurde Reinforced-Clostridial Agar verwendet. Das Nährmedium wurde in einem Autoklaven bei einem Überdruck von 80 bis 100 kPa für 20 bis 30 min sterilisiert. Nach Einstellung des pH-Wertes auf pH 6,6 bis 7,0 wurde das Medium in Glaspetrischalen gefüllt und erneut unter den selben Bedingungen sterilisiert. Die verfestigten Nährböden wurden auf Sterilität überprüft und mit Kulturen des *Propionibacterium acnes*-Impfstammes inokuliert. Der Impfstamm wurde auf der Oberfläche des Nährbodens verteilt und bei 35 bis 39°C für 46 bis 50 Stunden unter streng anaeroben Bedingungen kultiviert bis das Wachstum ein Optimum erreichte. Die Kulturen wurden in der Phase der höchsten Antigenproduktion

(bestimmt durch OD-Messunng bei 605 nm unter Verwendung eines Spektrometers, in Br) unter Verwendung von sterilem Wasser zur Injektion geerntet und bei 2 bis 8°C für 22 bis 26 Stunden extrahiert. Die erhaltene Zellmasse wurde durch Erhitzen auf 56 bis 62°C für eine Stunde dreimal in Abstand von mindestens 24 Stunden inaktiviert. Nach einem Test auf Sterilität wurde die inaktivierte Zellmasse für 48 Stunden lyophilisiert bis ein Restwassergehalt von bevorzugt unter 3% erhalten wurde.

Nach der Lyophilisierung wurden die Stämme in einem Träger aus einem Gemisch aus Aerosil, Saccharose und Kartoffelstärke oder einem anderen geeigneten Vehikel gemischt, sodass im Endprodukt die Stämme in einem Verhältnis a1 :a2:a3:a4 = 10:10:10:70 vorlagen.

Das so erhaltene Gemisch wurde in (Hart)kapsein zur oralen Applikation abgefüllt. Dabei enthielt jede Kapsel 2 mg Impfstämme und 248 mg TrägerNehikel.
Das Endprodukt wurde auf mikrobiologische Reinheit, Restwassergehalt, Löslichkeit und Formaldehydgehalt überprüft.

Bei der experimentellen Applikation an Mäusen induzierte der Impfstoff eine starke spezifische humorale und zelluläre Immunantwort wie unten gezeigt.

### Herstellungsbeispiel 2: Herstellung eines Impfstoffes zur vaginalen Applikation

Die zu verwendenden Stämme wurden gemäß der in Beispiel 1 beschriebenen Weise angezüchtet.
Nach der Lyophilisierung wurden die Stämme mit geschmolzener Kakaobutter gemischt, so dass im Endprodukt die Stämme in dem Verhältnis a1 :a2:a3:a4 = 15:15:15:55 vorlagen und Vaginalsuppositorien in bekannter Weise hergestellt. Ein Vaginalsuppositorium enthielt 6 mg Impfstämme (Trockengewicht) und 3 g Kakaobutter (oder eine andere Grundlage).
Das Endprodukt wurde auf mikrobiologische Reinheit, Löslichkeit und Formaldehydgehalt überprüft.
Die experimentelle Anwendung an Mäusen erbrachte ebenfalls die in Beispiel 1 gezeigten Eigenschaften.

### Herstellungsbeispiel 3: Herstellung eines Impfstoffes zur rektalen Applikation

Die zu verwendenden Stämme wurden gemäß der in Beispiel 1 beschriebenen Weise angezüchtet. Nach der Lyophilisierung wurden die Stämme mit geschmolzener Kakaobutter gemischt, sodass im Endprodukt die Stämme in dem Verhältnis a1:a2:a3:a4 = 20:20:20:40 vorlagen und Rektalsuppositorien in bekannter Weise hergestellt.

Ein Rektalsuppositorium enthielt 10 mg Impfstämme (Trockengewicht) und 3 g Kakaobutter (oder eine andere gebräuchliche Grundlage).
Das Endprodukt wurde auf mikrobiologische Reinheit, Löslichkeit und Formaldehydgehalt überprüft.
Die experimentelle Anwendung an Mäusen erbrachte ebenfalls die in Beispiel 1 und 2 gezeigten Eigenschaften.

### Herstellungsbeispiel 4: Herstellung des Impfstoffes durch Fermenter-technologie

Zur Anzucht der erfindungsgemäßen *Candida-* und *Propionibacterium acnes-*Stämme in großem Meßstab (250 bzw. 200 Liter Ansatz) wurde ein spezieller Bioreaktor mit kontrollierten Anzuchtbedingungen, wie z.B. APPLIKON ADI, 270 Liter, verwendet.

Die erfindungsgemäßen *Candida*-Impfstämme wurden separat in sterilem modifizierte Sabouraud-Medium mit einem pH-Wert von 5,8 bis 7,2 angezüchtet. Zur Inokulation des Nährmediums wurden frisch präparierte Kulturen des jeweiligen Candida-Stammes verwendet. Die Stämme wurden 16 bis 24 Stunden bei einer kontrollierten Temperatur von 23 bis 27°C unter aeroben Bedingungen kultiviert bis ein optimales Wachstum erreicht war (bestimmt durch OD-Messung bei 605 nm unter Verwendung eines Spektrometers, in Br).
Die Zellmasse wurde durch industrielle Ultrazentrifugation oder Ultrafiltration aus dem Medium isoliert.
Die weitere Vorgehensweise war identisch mit der in den Beispielen 1 bis 3 beschrieben statischen Methode unter der Verwendung von Agar.

Der *Propionibacterium acnes*-Stamm wurde in einem sterilen flüssigen modifizierten Reinforced-Clostridial-Medium ebenfalls in einem Bioreaktor bei einem pH-Wert von 6,4 bis 7,2 angezüchtet. Zur Inokulation des Nährmediums wurde eine frisch präparierte *Propionibacterium acnes*-Kultur verwendet. Die Kultivierung fand unter streng anaeroben Bedingungen bei einer kontrollierten Temperatur von 35 bis 39°C für 15 bis 17 Stunden statt. Die Zellmasse wurde durch industrielle Ultrazentrifugation oder Ultrafiltration isoliert.
Die weitere Vorgehensweise war identisch mit der in den Beispielen 1 bis 3 beschrieben statischen Methode unter der Verwendung von Agar.

### Nachweis der Wirksamkeit im Maus-Modell

### Beispiel 1: Immunstimulierende Wirkung des erfindungsgemäßen Impfstoffes

Eine Untersuchung der Leber- und Milzgewichte und des Gesamtgewichtes von Labormäusen welche mit *Propionibacterium acnes* CCM 7083, *Candida glabrata* CCM 8356, *Candida albicans* CCM 8355 und *Candida krusei* CCM 8357 immunisiert wurden im Vergleich mit nicht immunisierten Vergleichstieren wurde wie folgt durchgeführt um die immunstimulierende Wirkung des Impfstoffes zu zeigen.
**Impfung:** Dosis von 0,5 ml enthaltend 0.75 mg des *Propionibacterium acnes-*Stammes CCM 7083, 0,25 mg des *Candida glabrata*-Stammes CCM 8356, 0,25 mg des *Candida albicans* -Stammes CCM 8355 und 0,25 mg des *Candida krusei* -Stammes CCM 8357.
**Applikationsart:** intraperitoneal
**Versuchstierart:** weibliche weiße Labormäuse (outbreed HAN:ICR)
**Anzahl**: 110
**Gewicht.:** durchschnittlich 30 g (84 Tage alt)
**Anzahl der Tiere in den geimpften Gruppen: 11**
**Anzahl der Tiere in den Kontrollgruppen: 11**

**Methodik:** Töten und sezieren von jeweils einer immunisierten und einer nicht immunisierten (Kontroll) Gruppe von Mäusen erfolgt in regelmäßigen Abständen nach 0, 15, 30, 45 und 60 Tagen. Leber-, Milz- und das Gesamtgewicht wurde getrennt bestimmt. Der Versuch war 60 Tage nach der Verabreichung beendet. Nach 6 Wochen sollten die immunisierten Mäuse, wenn sie mit den nicht immunisierten Labormäusen verglichen werden wieder normal sein.

Nach der Verabreichung des *Propionibacterium acnes*-Stammes, des *Candida glabrata*-Stammes, des *Candida albicans* -Stammes und des *Candida krusei -* Stammes an die Mäuse zeigte sich eine signifikante Zunahme des Gewichts der Leber und der Milz im Vergleich zu den Kontrolltieren die keine Dosis der erfindungsgemäßen Stämme erhielten (siehe Tabelle 1, unten), was die immunstimulierende Wirkung des erfindungsgemäßen Impfstoffes zeigt.

Am 30. Tag nach der Verabreichung der Stämme war das Lebergewicht wieder normal, das Gewicht der Milz zeigte etwa am 45. Tag wieder normale Werte.

**Tabelle 1: Zusammenfassung der Ergebnisse von Beispiel 1**

| **Tage nach Verabreichung** | **Leber** | | **Milz** | | **Gesamtgewicht (g)** | |
|---|---|---|---|---|---|---|
| | **Immun. Maus** | **Kontroll Maus** | **Immun. Maus** | **Kontroll Maus** | **Immun. Maus** | **Kontroll Maus** |
| **0 (vor Verabreichung)** | - | - | - | - | - | - |
| **Tag 15** | + | - | + | - | - | - |
| **Tag 30** | - | - | + | - | - | - |
| **Tag 45** | - | - | - | - | - | - |
| **Tag 60** | - | - | - | - | - | - |

| | | | | | | |
|---|---|---|---|---|---|---|
| Erklärung: + signifikante Zunahme des Organgewichtes im Vergleich zu den Kontrolltieren - nicht signifikante Zunahme des Organgewichtes im Vergleich zu den Kontrolltieren | | | | | | |

### Beispiel 2: Challenge-Versuch unter Verwendung der Formulierung nach Herstellungsbeispiel 1

### Methode:

Vierzig C3H/HeJ-Mäuse mit einem Gewicht von 15 bis 18 g wurden für den Versuch verwendet. Die Mäuse wurden in vier Versuchsgruppen aufgeteilt:
- 1. Gruppe -10 Mäuse - weder Immunisierung noch Challenge
- 2. Gruppe von 10 Mäuse die mit der folgenden, der erfindungsgemäßen Formulierung für Kapseln zur humanen Verwendung (d.h. CANDIVAC Kapseln ad us. hum., Herstellungsbeispiel 1) in einer Dosis von 0,5 ml p.o. immunisiert wurden (eine Dosis pro Maus bedeutet 0,5 ml mit 1,5 mg gelösten Teststämmen- 0,75 mgdes *Propionibacterium acnes -* Stammes; 0,25 mg des *Candida albicans*-Stammes; 0,25 mg der *Candida glabrata*-Stammes und 0,25 mg des *Candida krusei-*Stammes). Der Impfstoff wurde den Tieren dieser Gruppe an den Testtagen 0, 7 und 14 verabreicht. Vierundzwanzig Stunden nach der letzten Dosis wurde den Tieren subkutan eine Dosis von 0,2 ml des Challenge-Bakterienstammes *Francisella tularensis,* enthaltend 1,5 x10^{4,0} Bakterien verabreicht (100 MLD₅₀).
- 3. Gruppe von 10 Mäusen- identisch mit Gruppe 2, jedoch wurde die Impfstoffpräparation intraperitoneal in einer Dosis von 0,5 ml verabreicht.
- 4. Gruppe von 10 Mäusen - Kontrollgruppe welcher nur der Challenge-Bakterienstamm *Francisella tularensis* in einer Dosis von 1,5 x 10^{4,0} (100 MLD₅₀) verabreicht wurde.

Nach dem Challenge mit *Francisella tularensis* wurden alle Tiere für 28 Tage beobachtet und die Morbidität und Mortalität der Versuchsmäuse wurde aufgezeichnet.

### Ergebnisse:

Der Impfstoff CANDIVAC Kapseln ad us. hum. gemäß dem Herstellungsbeispiel 1 erhöhte die Überlebensrate der Mäuse nach p.o. Verabreichung (80% der Mäuse überlebten) und nach i.p. Verabreichung (90% der Mäuse überlebte) signifikant gegenüber den nicht-immunisierten Kontrollmäusen (Mortalität von 100%), und gewährte somit Schutz.

Die Ergebnisse sind in Tabelle 2, unten zusammengefasst.

**Tabelle 2: Ergebnisse des Challenge-Versuchs:**

| **Gruppe Nr.** | **Anzahl der Mäuse** | **Immunisierung** | **Challenge *Francisella tularensis*** | **Anzahl der überlebend .Mäuse (Stck/ %)** | **Anzahl der toten Mäuse (Stck / %)** | **Mortalität** |
|---|---|---|---|---|---|---|
| 1 | 10 | - | - | 10 Stck (100 %) | 0 Stck (0 %) | Keine Toten |
| 2 | 10 | 0,5 ml p.o. | 100 MLD₅₀ | 8 Stck (80 %) | 2 Stck (20 %) | Tod der Mäuse am Tag 12 und 13 nach der Infektion |
| 3 | 10 | 0,5 ml i.p. | 100 MLD₅₀ | 9 Stck (90 %) | 1 Stck (10 %) | Tod am Tag 13 nach der Infektion |
| 4 | 10 | - | 100 MLD₅₀ | 0 Stck (0 %) | 10 Stck (100 %) | Tod zwischen Tag 7 und 11 nach der Infektion |

### Beispiel 3: Blasten-Transformationsversuch mit dem erfindungsgemäßen Impfstoff

### Versuchsmethode:

BALB/c Mäusen wurde eine Präparation, welche der erfindungsgemäßen Formulierung von Kapseln ad us. hum. (d.h. CANDIVAC Kapseln ad us. hum. gemäß Herstellungsbeispiel 1) entsprach, an den Tagen 0, 7 und 14, i.p. und s.c. verabreicht. Die Kontrollgruppe erhielt gepufferte Kochsalzlösung.
Ein Teil der Tiere wurde 24, 48, 72 Stunden, 5 und 7 Tage nach der letzten verabreichten Dosis getötet. Zellsuspensionen wurden aus den erhaltenen Milzen hergestellt und auf eine Konzentration von 4x10⁶ Zellen pro 1 ml Kulturmedium verdünnt. Jeweils 20 µl dieser Zellsuspension und 50 µl Mitogen wurden mit einer Pipette in der Vertiefungen des Testkits transferriert. ConA wurde in einer Konzentration von 5µl/1ml Medium und LPS (Lipopolysaccharid) in einer Konzentration von 20µl/1ml Medium verwendet. Die spezifischen Antigene des Impfstoffes CANDIVAC wurden in einer Konzentration von 100 µl/1ml Medium verwendet. Die Zellen und die Mitogene wurden für 67 Stunden bei einer Temperatur von 37°C bei 5% CO₂ inkubiert. Dann wurde 50µg markiertes Thymidin (1µ Ci pro Vertiefung) hinzugegeben. Die Testplatten wurden 5 weitere Stunden bei 37°C, 5% CO₂ und 100% Luftfeuchtigkeit inkubiert. Nach Ernte der Zellen mit einem Harvester Flow, wurden sie in Fläschen mit Szintilationsflüssigkeit überführt und die Radioaktivität (β-Strahlung) mit einem Rackbeta-Computer gemessen und in cpm ausgedrückt.

### Ergebnisse

Beide Verabreichungswege des Impfstoffes CANDIVAC verstärkt die Zellproliferation bei fast allen Zeitintervallen, nur am Tag 7 nach der p.o. Applikation ist diese statistisch nicht signifikant erhöht.

**Tabelle 3: Versuchsergebnisse des Blastentransformation von Milzzellen nach Verabreichung des erfindungsgemäßen Impfstoffes**

| Stimulation in vitro | Medium (PBS) | | ConA 5 µg/ml | | LPS 20 µg/ml | | CANDIVAC 100 µg/ml | |
|---|---|---|---|---|---|---|---|---|
| | per os | i.p. | per os | i.p. | per os | i.p. | per os | i.p. |
| Tag 1 | 3,18^{**} | 2,77^{**} | 0,97 | 1,77^{**} | 2,53^{**} | 0,52^{**} | 5,86^{**} | 4,24^{**} |
| Tag 2 | 1,89^{*} | 3.84^{**} | 1.23^{*} | 0,85^{*} | 1,27 | 0,57^{*} | 4.94^{**} | 5,32^{*} |
| Tag 3 | 2,67^{**} | 2.78^{**} | 0.71 | 0,39 | 1,63^{**} | 0,65^{*} | 2,33^{**} | 7,44^{**} |
| Tag 5 | 1,27 | 3,24^{*} | 0,72^{*} | 0,59^{*} | 1,34^{*} | 0,79 | 2,35^{**} | 8,53^{**} |
| Tag 7 | 1,87^{*} | 3,36 | 0,83 | 0,74 | 2,80^{**} | 0.54^{**} | 0,57 | 7.80^{**} |

| | | | | | | | | |
|---|---|---|---|---|---|---|---|---|
| ^{**}p < 0,01 ^{*}p < 0,05 | | | | | | | | |

Tabelle 3 zeigt die basale Lymphoproliferation von Milzzellen (als Medium bezeichnet) und die Proliferation von Spienocyten nach *in vitro* Stimulation mit ConA, LPS und dem erfindungsgemäßen Impfstoff. Die Ergebnisse sind als Transformationsindex gezeigt, dem Verhältnis der Lymphoproliferation von Zellen von immunisierten Mäusen und Kontrollmäusen.

### Bestimmung des erfindungsgemäßen Verhältnisses der einzelnen Stämme Candida albicans (CCM 8355), Candida glabrata (CCM 8356), Candida krusei (CCM 8357) und Propionibacterium acnes (CCM 7083)im erfindungsgemäßen Impfstoff im Tierversuch (Mäuse)

### Testmethode

Verschiedene Verhältnisse der erfindungsgemäßen Candida-Stämme und des erfindungsgemäßen *Propionibacterium-Stammes* wurden für die Herstellung eines Impfstoffes verwendet. 5 mg Trockensubstanz der Impfstämme in verschiedenen Verhältnissen wurden in 1ml in 1 ml PBS pH 7,2 gelöst.

Die so hergestellten Impfstoff-Zusammensetzungen wurden intraperitoneal SPF-ICR outbred weiblichen Mäusen mit einem Gewicht von 16 to 20 g (5 Mäuse, je 0,2 ml) verabreicht.
Die Tiere wurden nach 10 Tagen mit Chloroform getötet und seziert.
Die Gewichtszunahme von Leber und Milz wurde bestimmt und mittels Histologie untermauert. Eine Gruppe von 2 Mäusen, die nur PBS intraperitoneal erhielten dienten als Kontrollgruppe. Die Ergebisse der Gewichtszunahme wurden unter Verwendung eines nicht parametrischen U-Tests statitisch ausgewertet.

Die histologische Untersuchung von Leber und Milz erfolgte mittels einer Paraffinmethode und Anfärbung mit Haematoxilin und Eosin.

14 Tage nach der Immunisierung wurden die 3 verbleibenden immunisierten Tiere pro Gruppe und 3 nicht immunisierte Kontrolltiere mittels intravenöser Applikation von lebenden Candida albicans in einer Gesamtdosis von 10^{4.0} lebenden Candida-Zellen von Candida albicans pro Maus gechallenged. Alle Versuchstiere (immunisierte und nicht immunisierte Mäuse) wurden 4 Wochen nach dem Challenge-Versuch getötet und auf lebende Zellen der Candida albicans Stämme hin untersucht.

Der Impfstoff wird nur dann als schüzend angesehen, wenn kein Candida albicans Stamm aus den Nieren isoliert und kultiviert werden kann..

**Tabelle 4: Einfluss des Verhältnisses der Impfstämme Candida albicans (CCM 8355), Candida glabrata (CCM 8356), Candida krusei (CCM 8357) and Propionibacterium acnes (CCM 7083) (CA = a1, CG = a2, CK = a3, PA = a4) auf die Wirksamkeit und Sicherheit des daraus hergestellten Impfstoffes**

| Zusammensetzung des Impfstoffe | Staistisch signifikante (p≤ 0.05) Zunahme des Gewichts | | Histologischer immunmodulatori scher Effekt | | Schutzwirkung des Impfstoffes gegen *Candida albicans* | Unbedenklich keit | Ergebnis |
|---|---|---|---|---|---|---|---|
| | Leber | Milz | Leber | Milz | | | |
| 1. a1:a2:a2:a4 = 10-20:10-20:10-20:40-70 | + | + | + | + | + | unbedenklich | konform |
| 2. a1:a2:a2:a4 = 15-20:15-20:15 20:40-55 | + | + | + | + | + | unbedenklich | konform |
| 3. a1:a2:a2:a4 = 10-15:10-15:10-15:55-70 | + | + | + | + | + | unbedenklich | konform |
| 4. a1:a2:a2:a4 = 10:10:10:70 | + | + | + | + | + | unbedenklich | konform |
| 5. a1:a2:a2:a4 = 15:15:15:55 | + | + | + | + | + | unbedenklich | konform |
| 6. a1:a2:a2:a4 = 20:20:20:40 | + | + | + | + | + | unbedenklich | konform |
| 7. a1:a2:a2:a4 = 5:5:5:85 | + | + | + | + | - | unbedenklich | Nicht konform |
| 8. a1:a2:a2:a4 = 1:1:1:97 | + | + | + | + | - | unbedenklich | Nicht konform |
| 9 a1:a2:a2:a4 = 25:25:25:25 | - | + | - | + | - | unbedenklich | Nicht konform |
| 10. a1:a2:a2:a4 = 30:30:30:10 | - | - | - | - | - | unbedenklich | Nicht konform |

Die Ergenisse in Tabelle 4 zeigen, dass optimale Ergebnisse bezüglich der Wirksamkeit und der Unbedenklichkeit des Impfstoffes erzielt werden solange sich das Verhältnis der 4 Impfstämme (Candida albicans CCM 8355 = a1; Candida glabrata CCM 8356 = a2; Candida crusei CCM 8357 = a3; Propionibacterium acnes CCM 7083 = a4) erfindungsgemäss wie folgt verhält:
1. a1:a2:a3:a4 = 10-20:10-20:10-20:40-70
2. a1:a2:a3:a4 = 15-20:15-20:15-20:40-55
3. a1:a2:a3:a4 = 10-15:10-15:10-15:55-70
4. a1:a2:a3:a4 = 10:10:10:70
5. a1:a2:a3:a4 = 15:15:15:55
6. a1:a2:a3:a4 = 20:20:20:40

Die Impfstoff-Zusammensetzungen 7, 8, 9 und 10 zeigten keine Wirksamkeit im Challengetest mit lebendenden *Candida*-Stämmen.
Zusammensetzung 7 und 8 zeigten auf Grund der hohen Menge an *Propoionibacterium acnes* eine unspezifische Immunität. Ein spezfischer Schutz gegen *Candida* konnte nicht erzielt werden

Zusammensetzung 9 und 10 zeigten ein ungenügende Form der unspezifischen und spezifischen Immunität.

### Bestätigung der Wirksamkeit des erfindungsgemässen Impfstoffes aus den Stämmen Candida albicans (CCM 8355), Candida glabrata (CCM 8356), Candida krusei (CCM 8357), Propionibacterium acnes (CCM 7083) bei Frauen

**Tabelle 5a) Vaginale Applikationsform**

| **Ergebnis der Behandlung** | **Patienten anzahl** | **%** | **Beobachtungszeitraum (Monate)** | |
|---|---|---|---|---|
| | | | **Mittel** | **Bereich** |
| Exzellent | 6 | 54,5 | 9,7 | 2 -13 |
| Gut | 3 | 27,3 | 11,3 | 10-12 |
| Befriedigend | 0 | 0 | - | - |
| Indifferent | 2 | 18,2 | 12,0 | 12 |
| Gesamt | 11 | 100,0 | - | - |

**Tabelle 5b) Orale Applikationsform**

| **Ergebnis der Behandlung** | **Patienten anzahl** | **%** | **Beobachtungszeitraum (Monate)** | |
|---|---|---|---|---|
| | | | **Mittel** | **Bereich** |
| Exzellent | 24 | 58,5 | 10,2 | 7 - 16 |
| Gut | 9 | 21,9 | 11,0 | 7 - 16 |
| befriedigend | 4 | 9,8 | 7,0 | - |
| Indifferent | 4 | 9,8 | 7,3 | 2 - 11 |
| Gesamt | 41 | 100,0 | - | - |

**Tabelle 5c) Simultane Verabreichung von aginaler und oraler Applikationsform**

| **Ergebnis der Behandlung** | **Patienten anzahl** | **%** | **Beobachtungszeitraum (Monate)** | |
|---|---|---|---|---|
| | | | **Mittel** | **Bereich** |
| Exzellent | 5 | 35,7 | 8,6 | 6 -11 |
| Gut | 5 | 35,7 | 14,0 | 12 -18 |
| Befriedigend | 2 | 14,3 | 7,0 | 5 - 9 |
| Indifferent | 2 | 14,3 | 6,0 | 3 - 9 |
| Gesamt | 14 | 100,0 | - | - |

Die oben dargestellten Ergebnisse zeigen, dass der erfindungsgemäße Impfstoff bei dem Großteil der Patienten eine exzellente Wirksamkeit zeigte, was bei der Problematik einer *Candida*-Infektion, die sehr oft chronisch verläuft und systemisch werden kann und folglich sehr schwer zu behandeln ist, von besonderer Bedeutung ist.

### Anwendungsbeispiele in der Humanmedizin

### Anwendungsbeispiel 1: Vaginalsuppositorien

Eine 34 Jahre alte Frau litt nach einem Schwimmbadbesuch unter einer entzündlichen Vaginitis (Jucken, Brennen, Ausfluß), welche vermutlich durch Pilze verursacht wurde. Es wurde eine Behandlung mit Candida-Suppositorien nach Herstellungsbeispiel 2 durchgeführt - 5 Tage hintereinander abends je ein Zäpfchen vaginal. Nach einem Tag trat eine leichte Besserung der Beschwerden ein, nach 4 Tagen war die Patientin beschwerdefrei.

### Anwendungsbeispiel 2: Vaginalsuppositorien

Eine 47-jährige Patientin litt nach Antibiotika-Gabe wiederholt unter einer pilzbedingten Vaginitis. Die Behandlung mit 7 Suppositorien Candida-Vakzine nach Herstellungsbeispiel 2 an 5 aufeinanderfolgenden Tagen (abends, vaginal) führte zu einer Besserung nach 2 Tagen, nach 5 Tagen war die Patientin beschwerdefrei. Da weitere Antibiotika-Behandlungen absehbar waren, wurden 4 Wochen und 8 Wochen später noch einmal je 7 Kapseln eingenommen. Eine Antibiotika-Gabe nach 10 Wochen führte zu keinem Rezidiv der Vaginitis.

### Anwendungsbeispiel 3: Rektalsuppositorien

Ein 24 Jahre alter Patient litt unter starken Beschwerden durch Hämorrhoiden. Trotz Verödungsbehandlung waren immer noch Beschwerden durch Jucken und Brennen in der Analgegend vorhanden, die eine Pilzbesiedlung des Enddarmes vermuten ließen. Die Behandlung mit 10 Rektalsuppositorien nach Herstellungsbeispiel 3 (abends 10 Tage hintereinander rektal) führte nach 2-3 Tagen zu einer erheblichen Verbesserung der Beschwerden, die nach 6 Wochen noch fortbestehten

### Anwendungsbeispiel 4: Kapseln zur oralen Applikation

Ein 61-jähriger Patient litt aufgrund einer Leukopenie unter rezidivierendem Mundsoor - Ampho-Moronal (Amphothericin B) war aufgrund wiederholter Anwendung fast wirkungslos.
Die Einnahme von 5 Kapseln Candida-Vakzine gemäß Herstellungsbeispiel 1 führte.zu einer Verbesserung der Beschwerden nach 5 Tagen. Die Einnahme von 2 weiteren Zyklen mit je 5 Tagen - 4 und 8 Wochen später - hielt die Beschwerden trotz weiterbestehender Grunderkrankung in einem für den Patienten akzeptablen Bereich.

### Anwendungsbeispiel 5: Kapseln

Eine 72-jährige Patientin litt immer wieder unter unspezifischen Beschwerden im Magen-Darm-Bereich wie z.B. Stuhl-Unregelmäßigkeiten, Durchfall, Blähungen und Unwohlsein. Schwerwiegende organische Störungen wurden medizinisch ausgeschlossen, eine Besiedlung des Darmes mit Pilzen schien wahrscheinlich. Eine 3-mohatige Kur mit Candida-Kapseln gemäß Herstellungsbeispiel 1 (jeweils 10 Tage hintereinander am Monatsanfang, Einnahme von einer Kapsel täglich, morgens) führte bereits nach einem Zyklus zu einer erheblichen Verbesserung der Beschwerden, nach 3 Zyklen waren die Beschwerden weitgehend abgeklungen.

### Anwendungsbeispiele in der Veterinärmedizin

Der erfindungsgemäße Impfstoff ist zudem auch in der Veterinärmedizin verwendbar und wirksam. Besonders bei kleineren Tieren wie Katzen und Hunden wird seine Verwendung vorteilhaft sein.

Wie aus den obengemachten Ausführungen und Anwendungsbeispielen hervorgeht, stellt die vorliegende Erfindung somit einen hochwirksamen und zudem leicht zu applizierenden Impfstoff zur Immunoprophylaxe und Behandlung von Candidamykosen in der Veterinär- und Humanmedizin bereit. Der erfindungsgemäße Impfstoff ist in der Humanmedizin sowohl oral, parenteral als auch lokal, insbesondere vaginal als auch rektal, anwendbar. Besonders bevorzugt ist hierbei die orale, vaginale und rektale Anwendung. Ferner ist der erfindungsgemäße Impfstoff in der Veterinärmedizin parenteral, lokal oder oral anwendbar.

Somit wird ein einfach zu applizierender und gut verträglicher Impfstoff für die effektive Immunoprophylaxe und Therapie von Candidamykosen für die Anwendung in der Human- und Veterinärmedizin bereitgestellt. Durch die bevorzugte orale oder lokale Applikation wird zudem das Risiko einer eventuellen anaphlyaktischen oder anaphylaktoiden Reaktion ausgeschlossen.

## Patentansprüche

1. Impfstoff bestehend aus der Kombination der Candida-Stämme
a1) *Candida albicans* CCM 8355,
a2) *Candida glabrata* CCM 8356,
a3) *Candida krusei* CCM 8357 und
a4) dem immunmodulierenden *Propionibacterium acnes*-Stamm *Propionibacterium acnes* CCM 7083,
und gegebenenfalls einem oder mehrer Hilfsstoffe,
wobei das Verhältnis der Bestandteile a1 - a4 im Endprodukt a1:a2:a3:a4 = 10-20: 10-20: 10-20 : 40-70 beträgt.

2. Impfstoff nach Anspruch 1,
wobei das Verhältnis a1:a2:a3:a4 = 15-20:15-20:15-20:40-55 ist.

3. Impfstoff nach Anspruch 1,
wobei das Verhältnis a1:a2:a3:a4 = 10-15:10-15:10-15:55-70 ist.

4. Impfstoff nach einem der vorstehenden Ansprüche,
wobei das Gesamttrockengewicht aller Impfstämme a1 bis a4 2 bis 10 mg pro zu applizierende Dosis beträgt.

5. Impfstoff nach einem der vorstehenden Ansprüche,
wobei der Gesamtgehalt an Formaldehyd im Endprodukt weniger als 0,02 Gew. % beträgt.

6. Impfstoff nach einem der vorstehenden Ansprüche zur parenteralen, oralen oder lokalen Applikation.

7. Impfstoff nach Anspruch 6 zur topischen, vaginalen oder rektalen Applikation.

8. Impfstoff nach Anspruch 6 zur oralen Applikation,
wobei das Verhältnis a1:a2:a3:a4 = 10:10:10:70 beträgt.

9. Impfstoff nach Anspruch 6 zur vaginalen Applikation,
wobei das Verhältnis a1:a2:a3:a4 = 15:15:15:55 beträgt.

10. Impfstoff nach Anspruch 6 zur rektalen Applikation,
wobei das Verhältnis a1:a2:a3:a4 = 20:20:20:40 beträgt.

11. Impfstoff nach einem der vorstehenden Ansprüche formuliert in Form von Kapseln,Tabletten, Lutschtabletten, Pastillen, Sirupen, oralen Suspensionen, oralen Emulsionen, Globuli, Pillen, Suppositorien, Vaginalovula, Ampullen, Fertigspritzen, Aerosolen, Insufflationen oder Mundwassern.

12. Impfstoff nach einem der vorstehenden Ansprüche zur Verwendung in der Prophylaxe und/oder Behandlung von lokaler, kutaner oder mukokutaner und/oder systemischer Candidiasis.

13. Impfstoff nach Anspruch 12,
wobei die lokale Candidiasis die äußere Mucosa des Genitaltraktes, den Urogenitaltrakt, die Mundhöhle, den Gastrointestinaltrakt, die Brustdrüsen, den Gehörgang oder die Haut betrifft.

14. Impfstoff nach Anspruch 13 zur Verwendung in der Prophylaxe und/oder Behandlung von Stomatitis (Soor), Ösophagitis, Windelerythem oder Vulvovaginitis.

15. Impfstoff nach Anspruch 12,
wobei die systemische Candidiasis ausgewählt ist aus generalisierter systemischer Candidose, Candidämie, akuter hämatogen disseminierter Candidiasis, chronisch disseminierter Candidiasis, Candida-Endokarditis, Candida-Perikarditis, eitrige Phlebitis, Candida-Meningitis, Candida-Pneumonie, Candida-Osteomyelitis, Candida-Mediastinitis, Candida-Arthritis, oropharyngealer und ösophagealer Candidiasis.

16. Impfstoff nach einer der Ansprüche 1 bis 15 zur Anwendung in der Veterinärmedizin.

17. Impfstoff nach einem der Ansprüche 1 bis 15 zur Anwendung in der Humanmedizin.

18. Verfahren zur Herstellung des Impfstoffes nach einem der Ansprüche 1 bis 17, umfassend
1) das getrennte Heranzüchten der Impfstämme a1 bis a4;
2) Inaktivieren der Candida-Stämme a1 bis a3 mittels Formaldehyd;
3) Hitzeinaktivieren des *Propionibacterium*-Stammes a4;
4) Lyophilisation der inaktivierten Stämme; und
5) Vermischen der Impfstämme mit gegebenenfalls einem oder mehreren Hilfsstoffen, um im Endprodukt ein Verhältnis von a1:a2:a3:a4 = 10-20 : 10-20 : 10-20 : 40-70 zu erhalten,
wobei in Schritt 1) die Candida-Stämme a1 bis a3 auf Glucosopepton-Agar oder Sabouraud-Agar bei 23 bis 27° C für 48 bis 72 Stunden oder bei 35 bis 39° C für 22 bis 27 Stunden bei aeroben Bedingungen unter Normaldruck und 90 bis 98 % Luftfeuchtigkeit angezüchtet werden und dann mit sterilem Wasser zur Injektion gewaschen werden und durch drei Gefrier- und Tauzyklen desintegriert werden oder wobei in Schritt 1) die Candida-Stämme a1 bis a3 in einem Sabouraud-Medium bei 37°C für 24 bis 48 Stunden unter aeroben Bedingungen herangezüchtet werden, danach die Bakterienzellen unter Verwendung einer Ultrafiltrationspatrone mit einem Cut-off von 300 kDa isoliert werden, gefolgt von Reinigung durch wiederholtes Waschen mit steriler physiologischer Kochsalzlösung und Zentrifugation bei 4500 g, gefolgt von Waschen mit sterilem Wasser zur Injektion und Desintegration der Bakterienzellen durch drei Gefrier- und Tauzyklen und wobei in Schritt 1) der Propionibacterium acnes-Stamm a4 auf einem Blutagar, einem Reinforced-Closstridial-Agar oder einem VL-Agar mit Blut bei 35 bis 39° C für 46 bis 50 Stunden unter streng anaeroben Bedingungen bei Normaldruck und einer Luftfeuchtigkeit von 90 bis 98 % herangezüchtet wird, gefolgt von Lyse der Bakterien mit sterilem destilliertem Wasser und Extraktion bei 2 bis 8° C für 22 bis 26 Stunden, und in Schritt 3) das bakterielle Material durch dreimaliges Erhitzen auf 56 bis 62° C für mindestens eine Stunde in einem Abstand von mindestens 24 Stunden inaktiviert wird oder wobei in Schritt 1) der Propionibacterium acnes-Stamm a4 in einem Reinforced-Clostridial-Medium bei 35 bis 39° C für 46 bis 50 Stunden unter streng anaeroben Bedingungen unter statischen Bedingungen herangezüchtet wird, die Bakterienzellen unter Verwendung einer Ultrafiltrationspatrone mit einem Cut-off von 300 kDa isoliert werden, gefolgt von Reinigung durch wiederholtes Waschen mit steriler physiologischer Kochsalzlösung und Zentrifugation bei 4500 g, und gefolgt von Lyse der Bakterien mit sterilem destilliertem Wasser und Extraktion bei 2 bis 8° C für 22 bis 26 Stunden, und in Schritt 3) das bakterielle Material durch dreimaliges Erhitzen auf 56 bis 62° C für mindestens eine Stunde in einem Abstand von mindestens 24 Stunden inaktiviert wird.

19. Verfahren nach Anspruch 18, wobei das Gesamttrockengewicht aller Impfstämme 2 bis 10 mg pro Dosis ist.

20. Verfahren nach Anspruch 18 oder 19, wobei der Formaldehydgehalt im Endprodukt weniger als 0,02 Gew. % beträgt.

21. Verfahren nach einem der Ansprüche 18 bis 20, wobei die Impfstämme a1 bis a4 großtechnisch in Fermenten kultiviert werden.

22. Verfahren nach Anspruch 21, wobei in Schritt 1) die Candida Stämme a1-a3 in einem Sabouraud Medium mit einem pH-Wert von 5,6 bis 7,2 bei einer Temperatur von 23-27°C für 16 bis 24 Stunden unter aeroben Bedingungen mit 15-20 % gelöstem Sauerstoff bei einem Druck von 0,1 bis 0,2 bar gegenüber dem atmosphärischem Druck unter Zugabe von gefilterter Umgebungsluft bei 40 bis 50 UPM angezüchtet werden und die Stämme mittels industrieller Ultrazentrifugation bei 3600 bis 5300 g, Ultrafiltration mit einer Ultrafiltrationspatrone mit einem Cut-off von 300 kDa oder einer Kombination davon isoliert werden.

23. Verfahren nach Anspruch 21 oder 22, wobei in Schritt 1) der Propionibacterium acnes Stamm in Reinforced-Clostridial-Medium mit einem pH-Wert von 6,4 bis 7,2 bei einer Temperatur von 35-39° C für 15 bis 17 Stunden unter streng anaeroben Bedingungen mit einem Maximum von 1 % gelöstem Sauerstoff bei einem Druck von 0,1 bis 0,2 bar gegenüber dem atmosphärischem Druck unter der Zugabe von 10 Liter einer gefilterten Mischung von N:CO2 (1:2) pro Minute bei 40 bis 50 UPM angezüchtet werden und der Stamm mittels industrieller Ultrazentrifugation bei 3600 bis 5300 g, Ultrafiltration mit einer Ultrafiltrationspatrone mit einem Cut-off von 300 kDa oder einer Kombination davon isoliert wird.

## Claims

1. A vaccine, comprixing the combination of the following Candida strains
a1) *Candida albicans* CCM 8355,
a2) *Candida glabrata* CCM 8356,
a3) *Candida krusei* CCM 8357 and
a4) the immunomodulating *Propionibacterium acnes* strain *Propionibacterium acnes* CCM 7083
and optionally one or more adjuvants,
wherein the ratio of the ingredients a1 through a4 in the final product is a1:a2:a3:a4 = 10-20:10-20:10-20:40-70.

2. The vaccine as defined by claim 1,
wherein the ratio is a1:a2:a3:a4 = 15-20:15-20:15-20:40-55.

3. The vaccine as defined by claim 1,
wherein the ratio is a1:a2:a3:a4 = 10-15:10-15:20-25:55-70.

4. The vaccine as defined by one of the foregoing claims,
wherein the total dry weight of all the vaccine strains a1 through a4 is from 2 to 10 mg per dose to be administered.

5. The vaccine as defined by one of the foregoing claims,
wherein the total formaldehyde content in the final product is less than 0.02 weight %.

6. The vaccine as defined by one of the foregoing claims for parenteral, oral or local administration.

7. The vaccine as defined by claim 6 for topical, vaginal or rectal administration.

8. The vaccine as defined by claim 6 for oral administration,
wherein the ratio is a1:a2:a3:a4 = 10:10:10:70.

9. The vaccine as defined by claim 6 for vaginal administration,
wherein the ratio is a1:a2:a3:a4 = 15:15:15:55.

10. The vaccine as defined by claim 6 for rectal administration,
wherein the ratio a1:a2:a3:a4 = 20:20:20:40.

11. The vaccine as defined by one of the foregoing claims, formulated in the form of capsules, tablets, drops to suck on, lozenges, syrups, oral suspensions, oral emulsions, globules, pills, suppositories, vaginal ovulas, ampules, prefilled syringes, aerosols, insufflations, or mouthwashes.

12. The vaccine as defined by one of the foregoing claims for use in the prophylaxis and/or treatment of local, cutaneous, or mucocutaneous and/or systemic candidiasis.

13. The vaccine as defined by claim 12,
wherein the local candidiasis concerns the external mucosa of the genital tract, the urogenital tract, the oral cavity, the gastrointestinal tract, the mammary glands, the auditory canal, or the skin.

14. The vaccine as defined by claim 13 for use in the prophylaxis and/or treatment of stomatitis (thrush), esophagitis, diaper rash, or vulvovaginitis.

15. The vaccine as defined by claim 12,
wherein the systemic candidiasis is selected from the group comprising generalized systemic candidiasis, candidemia, acute hematogenically disseminated candidiasis, chronicly disseminated candidiasis, candidial endocarditis, candidial pericarditis, suppurative phlebitis, candidial meningitis, candidial pneumonia, candidial osteomyelitis, candidial mediastinitis, candidial arthritis, and oropharyngeal and esophageal candidiasis.

16. The vaccine as defined by one of claims 1-15 for use in veterinary medicine.

17. The vaccine as defined by one of claims 1-15 for use in human medicine.

18. A method for producing the vaccine defined by one of claims 1-17, including
1) the separate cultivation of the Candida strains a1 through a4;
2) deactivation of the Candida strains a1 through a3 by means of formaldehyde;
3) heat deactivation of the *Propionibacterium* strain a4;
4) lyophilization of the deactivated strains;
5) mixing of the vaccine strains with optionally one or more adjuvants, in order to attain a ratio in the final product of a1:a2:a3:a4 = 10-20:10-20:10-20:40-70,
wherein in step 1), the Candida strains a1 through a3 are cultured on glucose peptone agar or Sabouraud agar at 23 to 27°C for 48 to 72 hours or at 35 to 39°C for 22 to 27 hours under aerobic conditions at standard pressure and 90 to 98% humidity and then washed with sterile water for injection and disintegrated by means of three cycles of freezing and thawing; or wherein in step 1) the Candida strains a1 through a3 are cultured in a Sabouraud medium at 37°C for 24 to 48 hours under aerobic conditions, after which the bacterial cells are isolated using an ultrafiltration cartridge with a cutoff of 300 kDa, followed by cleaning by repeated washing with sterile physiological saline solution and centrifuging at 4500 g, followed by washing with sterile water for injection and disintegration of the bacterial cells by means of three cycles of freezing and thawing; and wherein in step 1) the Proprionibacterium acnes strain a4 is cultured on a blood agar, a reinforced clostridial agar, or a VL agar with blood at 35 to 39°C for 46 to 50 hours under stringently anaerobic conditions at standard pressure and at a humidity of 90 to 98%, followed by lysis of the bacteria with sterile distilled water and extraction at 2 to 8°C for 22 to 26 hours, and in step 3) the bacterial material is deactivated by being heated three times to from 56 to 62°C for at least one hour at an interval of at least 24 hours; or wherein in step 1) the Propionibacterium acnes strain a4 is cultured in a reinforced clostridial medium at 35 to 39°C for 46 to 50 hours under stringently anaerobic conditions under static conditions, the bacterial cells are isolated using an ultrafiltration cartridge with a cutoff of 300 kDa, followed by cleaning by repeated washing with sterile physiological saline solution and centrifuging at 4500 g, and followed by lysis of the bacteria with sterile distilled water and extraction at 2 to 8°C for 22 to 26 hours, and in step 3) the bacterial material is deactivated by being heated three times to from 56 to 62°C for at least one hour at an interval of at least 24 hours.

19. The method as defined by claim 18, wherein the total dry weight of all the vaccine strains is from 2 to 10 mg per dose.

20. The method as define by claim 18 or 19, wherein the formaldehyde content in the final product is less than 0.02 weight %.

21. The method as defined by one of claims 18-20, wherein the vaccine strains a1 through a4 are cultivated on a large scale in ferments.

22. The method as defined by claim 21, wherein in step 1) the Candida strains a1-a3 are cultured in a Sabouraud medium having a pH of 5.6 to 7.2 at a temperature of from 23 to 27°C for 16 to 24 hours under aerobic conditions with from 15 to 20% dissolved oxygen at a pressure of from 0.1 to 0.2 bar compared with atmospheric pressure, with the addition of filtered ambient air at 40 to 50 rpm, and the strains are isolated by means of industrial ultracentrifuging at 3600 to 5300 g, ultrafiltration with an ultrafiltration cartridge with a cutoff of 300 kDa, or a combination thereof.

23. The method as defined by claim 21 or 22, wherein in step 1 the Priopionibacterium acnes strain is cultured in reinforced clostridial medium having a pH of 6.4 to 7.2 at a temperature of 35 to 39°C for 15 to 17 hours under stringently anaerobic conditions with a maximum of 1% dissolved oxygen at a pressure of 0.1 to 0.2 bar compared to atmospheric pressure, with the addition of 10 liters of a filtered mixture of N:CO2 (1:2) per minute at 40 to 50 rpm, and the strain is isolated by means of industrial ultracentrifuging at 3600 to 5300 g, ultrafiltration with an ultrafiltration cartridge with a cutoff of 300 kDa, or a combination thereof.

## Revendications

1. Vaccin composé d'une combinaison des souches Candida
a1) *Candida albicans* CCM 8355,
a2) *Candida glabrata* CCM 8356,
a3) *Candida krusei* CCM 8357 et
a4) la souche *Propionibacterium acnes* immunomodulante *Propionibacterium acnes* CCM 7083,
et éventuellement d'un ou de plusieurs adjuvants, où le rapport des constituants a1 à a4 dans le produit final est de a1:a2:a3:a4 = 10-20:10-20:10-20:40-70.

2. Vaccin selon la revendication 1, dans lequel le rapport est de a1:a2:a3:a4 = 15-20:15-20:15-20:40-55.

3. Vaccin selon la revendication 1, dans lequel le rapport est de a1:a2:a3:a4 = 10-15:10-15:10-15:55-70.

4. Vaccin selon l'une des revendications précédentes, dans lequel le poids sec total de toutes les souches vaccinales a1 à a4 est compris entre 2 et 10 mg par dose à appliquer.

5. Vaccin selon l'une des revendications précédentes, dans lequel la teneur totale en formaldéhyde dans le produit final est inférieure à 0,02 % en poids.

6. Vaccin selon l'une des revendications précédentes destiné à une application parentérale, orale ou locale.

7. Vaccin selon la revendication, 6 destiné à une application topique, vaginale ou rectale.

8. Vaccin selon la revendication 6 destiné à une application orale, dans lequel le rapport a1:a2:a3:a4 = 10:10:10:70.

9. Vaccin selon la revendication 6 destiné à une application vaginale, dans lequel le rapport a1:a2:a3:a4 = 15:15:15:55.

10. Vaccin selon la revendication 6 destiné à une application rectale, dans lequel le rapport a1:a2:a3:a4 = 20:20:20:40.

11. Vaccin selon l'une quelconque des revendications précédentes, formulé sous la forme de capsules, de comprimés, de comprimés à sucer, de pastilles, de sirops, de suspensions orales, d'émulsions orales, de granules, de pilules, de suppositoires, d'ovules vaginaux, d'ampoules, de seringues préremplies, d'aérosols, d'insufflations ou de collutoires.

12. Vaccin selon l'une des revendications précédentes, destiné à une utilisation dans la prophylaxie et/ou le traitement de candidose locale, cutanée ou muco-cutanée et/ou systémique.

13. Vaccin selon la revendication 12, dans lequel la candidose locale concerne la muqueuse externe du tractus génital, le tractus urogénital, la cavité buccale, le tractus gastro-intestinal, les glandes mammaires, le conduit auditif ou la peau.

14. Vaccin selon la revendication 13, destiné à une utilisation dans la prophylaxie et/ou le traitement d'une stomatite (muguet), d'une oesophagite, d'un érythème fessier ou d'une vulvovaginite.

15. Vaccin selon la revendication 12, dans lequel la candidose systémique est sélectionnée parmi une candidose systémique, une candidémie, une candidose disséminée hématogène aiguë, une candidose disséminée chronique, une endocardite à Candida, une péricardite à Candida, une phlébite purulente, une méningite à Candida, une pneumonie à Candida, une ostéomyélite à Candida, une médiastinite à Candida, une arthrite à Candida, une candidose oropharyngienne et oesophagienne.

16. Vaccin selon l'une des revendications 1 à 15, destiné à une application en médecine vétérinaire.

17. Vaccin selon l'une des revendications 1 à 15, destiné à une application en médecine humaine.

18. Procédé de préparation du vaccin selon l'une des revendications 1 à 17, comprenant
1) la culture séparée des souches vaccinales a1 à a4 ;
2) l'inactivation des souches Candida a1 à a3 au moyen de formaldéhyde ;
3) l'activation à la chaleur de la souche *Propionibacterium* a4 ;
4) la lyophilisation des souches inactivées ; et
5) le mélange des souches vaccinales avec éventuellement un ou plusieurs adjuvants pour obtenir dans le produit final un rapport de a1:a2:a3:a4 = 10-20:10-20:10-20:40-70.
dans lequel à l'étape 1), les souches Candida a1 à a3 sont cultivées sur glucosopeptone-gélose ou gélose de Sabouraud entre 23 et 27 °C pendant 48 à 72 heures ou entre 35 et 39 °C pendant 22 à 27 heures, dans des conditions aérobies, sous pression normale et avec 90 à 98 % d'humidité de l'air, puis lavées avec de l'eau stérile pour injection et désintégrées par des cycles de congélation / décongélation, ou dans lequel à l'étape 1) les souches Candida a1 à a3 sont cultivées dans un milieu de Sabouraud à 37 °C pendant 24 à 48 heures dans des conditions aérobies, puis les cellules bactériennes sont isolées en utilisant un module d'ultrafiltration avec seuil de coupure de 300 kDa, puis suit une purification par lavage répété avec un sérum physiologique stérile et une centrifugation à 4500 g, puis un lavage avec de l'eau stérile pour injection et une désintégration des cellules bactériennes par des cycles de congélation-décongélation, et dans lequel à l'étape 1) la souche Propionibacterium acnes a4 est cultivée sur une gélose au sang, une gélose clostridiale renforcée ou une gélose VL avec sang entre 35 et 39 °C pendant 46 à 50 heures dans des conditions anaérobies strictes, sous pression normale et avec une humidité de l'air de 90 à 98 %, puis suit une lyse des bactéries avec de l'eau distillée stérile et une extraction entre 2 et 8 °C pendant 22 à 26 heures, et à l'étape 3) le matériau bactérien est inactivé par trois chauffages à 56 à 62 °C pendant au moins une heure, avec un écart ou espacement d'au moins 24 heures, ou dans lequel à l'étape 1) la souche Propionibacterium acnes a4 est cultivée dans un milieu clostridial renforcé entre 35 et 39 °C pendant 46 à 50 heures dans des conditions anaérobies strictes et dans des conditions statiques, les cellules bactériennes sont isolées en utilisant un module d'ultrafiltration avec seuil de coupure de 300 kDa, puis suit une purification par un lavage répété avec du sérum physiologique stérile et une centrifugation à 4500 g, suivie d'une lyse des bactéries avec de l'eau distillée stérile et d'une extraction entre 2 et 8 °C pendant 22 à 26 heures, et à l'étape 3), le matériau bactérien est inactivé par trois chauffages entre 56 et 62 °C pendant au moins une heure, avec un écart ou espacement d'au moins 24 heures.

19. Procédé selon la revendication 18, dans lequel le poids sec total de toutes les souches vaccinales est compris entre 2 et 10 mg par dose.

20. Procédé selon la revendication 18 ou 19, dans lequel la teneur en formaldéhyde dans le produit final est inférieure à 0,02 % en poids.

21. Procédé selon l'une des revendications 18 à 20, dans lequel les souches vaccinales a1 à a4 sont cultivées à l'échelle industrielle dans des fermenteurs.

22. Procédé selon la revendication 21, dans lequel, à l'étape 1), les souches Candida a1 à a3 sont cultivées dans un milieu de Sabouraud ayant une valeur de pH comprise entre 5,6 et 7,2, à une température comprise entre 23 et 27 °C pendant 16 à 24 heures dans des conditions aérobies, avec 15 à 20 % d'oxygène dissout, à une pression comprise entre 0,1 et 0,2 bar par rapport à la pression atmosphérique, en ajoutant de l'air ambiant filtré, à 40 à 50 tpm, et les souches sont isolées au moyen d'une ultracentrifugation industrielle entre 3600 et 5300 g, d'une ultrafiltration avec un module d'ultrafiltration avec seuil de coupure de 300 kDa, ou d'une combinaison de ceux-ci.

23. Procédé selon la revendication 21 ou 22, dans lequel à l'étape 1), la souche Propionibacterium acnes est cultivée dans un milieu de clostridial renforcé ayant une valeur de pH comprise entre 6,4 et 7,2, à une température comprise entre 35 et 39 °C pendant 15 à 17 heures dans des conditions anaérobies strictes, avec un maximum de 1 % d'oxygène dissous, à une pression comprise entre 0,1 et 0,2 bar par rapport à la pression atmosphérique, en ajoutant 10 litres d'un mélange filtré de N:CO2 (1 :2) par minute à 40 à 50 tpm, et la souche est isolée au moyen d'une ultracentrifugation industrielle entre 3600 et 5300 g, d'une ultrafiltration avec un module d'ultrafiltration avec seuil de coupure de 300 kDa ou d'une combinaison de ceux-ci.
